Europäisches Patentamt

European Patent Office

Office européen des brevets

(11) Publication number: **0 481 673 A2**

## EUROPEAN PATENT APPLICATION

(21) Application number: **91309299.5**

(22) Date of filing: **09.10.91**

(51) Int. Cl.5: **C12N 15/61**, C12N 9/90, C12N 1/21, C12Q 1/533, //(C12N9/90,C12R1:865)

(30) Priority: **15.10.90 US 597645**

(43) Date of publication of application:
**22.04.92 Bulletin 92/17**

(84) Designated Contracting States:
**CH DE FR GB IT LI NL**

(71) Applicant: **MERCK & CO. INC.**
**126, East Lincoln Avenue P.O. Box 2000**
**Rahway New Jersey 07065-0900(US)**

(72) Inventor: **Siekierka, John J.**
**10 Glenview Drive**
**Towaco, NJ 07082(US)**
Inventor: **Wiederrecht, Gregory J.**
**25B Duncan Hill**
**Westfield, NJ 07090(US)**

(74) Representative: **Thompson, John Dr.**
**Merck & Co., Inc. European Patent**
**Department Terlings Park Eastwick Road**
**Harlow, Essex CM20 2OR(GB)**

(54) Genes encoding the human FK-506 binding protein and a S. cerevisiae homolog, FKB1, and their expression.

(57) The genes encoding the human FK-506 binding protein and a S. cerevisiae homolog, FKB1, are described, as well as their expression and the amino acid sequences of the protein products. These binding proteins are useful, inter alia, for screening compounds useful as immunosuppressive drugs that block allograft rejection by preventing T-cell activation.

EP 0 481 673 A2

FIGURE 2

```
                                    -10
                           GCGTC CGCCGGCGCC

1   ATG GGA GTG CAG GTG GAA ACC ATC TCC CCA GGA GAC GGG CGC ACC
    MET GLY VAL GLN VAL GLU THR ILE SER PRO GLY ASP GLY ARG THR

50  TTC CCC AAG CGC GGC CAG ACC TGC GTG GTG CAC TAC ACC GGG ATG
    PHE PRO LYS ARG GLY GLN THR CYS VAL VAL HIS TYR THR GLY MET

100 CTT GAA GAT GGA AAG TTT AAA TTT GAT TCC CGG GAC AGA AAC AAG
    LEU GLU ASP GLY LYS PHE LYS PHE ASP SER ARG ASP ARG ASN LYS

150 CCC TTT AAG TTT ATG CTA GGC AAG CAG GTG ATC CGA GGC TGG
    PRO PHE LYS PHE MET LEU GLY LYS GLN VAL ILE ARG GLY TRP

200 GAA GAA GGG GTT GCC CAG AGT GTG GGT CAG AGA GCC AAA CTG
    GLU GLU GLY VAL ALA GLN SER VAL GLY GLN ARG ALA LYS LEU

250 ACT ATA TCT CCA GAT TAT GCC TAT GGT GCC ACT GGG CAC CCA GGC
    THR ILE SER PRO ASP TYR ALA TYR GLY ALA THR GLY HIS PRO GLY

300 ATC ATC CCA CCA CAT GCC ACT CTC GTC TTC GAT GTG GAG CTT CTA
    ILE ILE PRO PRO HIS ALA THR LEU VAL PHE ASP VAL GLU LEU LEU
```

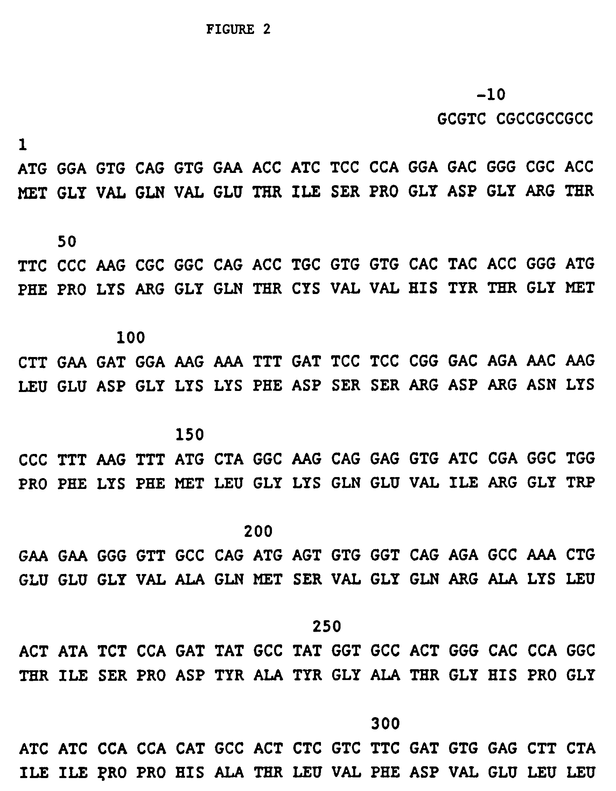

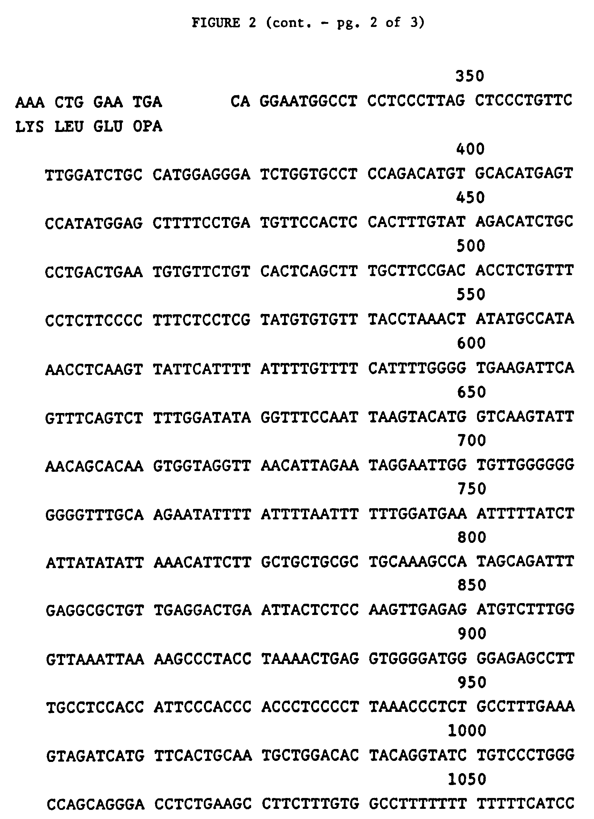

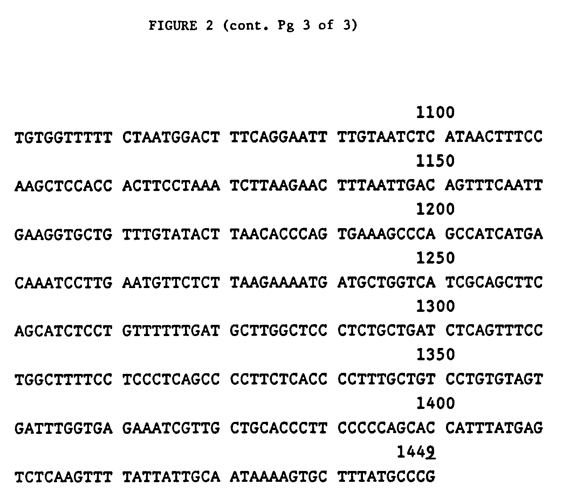

FIGURE 2 (cont. - pg. 2 of 3)

```
                                    350
AAA CTG GAA TGA       CA GGAATGGCCT CCTCCCTTAG CTCCCTGTTC
LYS LEU GLU OPA
                                    400
TTGGATCTGC CATGGAGGGA TCTGGTGCCT CCAGACATGT GCACATGAGT
                                    450
CCATATGGAG CTTTTCCTGA TGTTCCACTC CACTTTGTAT AGACATCTGC
                                    500
CCTGACTGAA TGTGTTCTGT CACTCAGCTT TGCTTCCGAC ACCTCTGTTT
                                    550
CCTCTTCCCC TTTCTCCTCG TATGTGTGTT TACCTAAACT ATATGCCATA
                                    600
AACCTCAAGT TATTCATTTT ATTTTGTTTT CATTTTGGGG TGAAGATTCA
                                    650
GTTTCAGTCT TTTGGATATA GGTTTCCAAT TAAGTACATG GTCAAGTATT
                                    700
AACAGCACAA GTGGTAGGTT AACATTAGAA TAGGAATTGG TGTTGGGGGG
                                    750
GGGGTTTGCA AGAATATTTT ATTTTAATTT TTTGGATGAA ATTTTTATCT
                                    800
ATTATATATT AAACATTCTT GCTGCTGCGC TGCAAAGCCA TAGCAGATTT
                                    850
GAGGCGCTGT TGAGGACTGA ATTACTCTCC AAGTTGAGAG ATGTCTTTGG
                                    900
GTTAAATTAA AAGCCCTACC TAAAACTGAG GTGGGGATGG GGAGAGCCTT
                                    950
TGCCTCCACC ATTCCCACCC ACCCTCCCCT TAAACCCTCT GCCTTTGAAA
                                    1000
GTAGATCATG TTCACTGCAA TGCTGGACAC TACAGGTATC TGTCCCTGGG
                                    1050
CCAGCAGGGA CCTCTGAAGC CTTCTTTGTG GCCTTTTTTT TTTTTCATCC
```

FIGURE 2 (cont. Pg 3 of 3)

```
                                    1100
TGTGGTTTTT CTAATGGACT TTCAGGAATT TTGTAATCTC ATAACTTTCC
                                    1150
AAGCTCCACC ACTTCCTAAA TCTTAAGAAC TTTAATTGAC AGTTTCAATT
                                    1200
GAAGGTGCTG TTTGTATACT TAACACCCAG TGAAAGCCCA GCCATCATGA
                                    1250
CAAATCCTTG AATGTTCTCT TAAGAAAATG ATGCTGGTCA TCGCAGCTTC
                                    1300
AGCATCTCCT GTTTTTTGAT GCTTGGCTCC CTCTGCTGAT CTCAGTTTCC
                                    1350
TGGCTTTTCC TCCCTCAGCC CCTTCTCACC CCTTTGCTGT CCTGTGTAGT
                                    1400
GATTTGGTGA GAAATCGTTG CTGCACCCTT CCCCCAGCAC CATTTATGAG
                                    1449
TCTCAAGTTT TATTATTGCA ATAAAAGTGC TTTATGCCCG
```

BACKGROUND OF THE INVENTION

1. Field of the Invention

The field of the present invention is immunosuppressive drugs that block allograft rejection by preventing T-cell activation.

FK-506, a neutral macrolide metabolite of S. tsukabaensis, is an important new immunosuppressive drug with 10 to 100 times the potency of the cyclic peptide, cyclosporin A (CsA), as measured by its efficacy in preventing allograft rejection. Both FK-506 and CsA exert their inhibitory effects during the signal transduction events leading to T-lymphocyte activation by selectively blocking transcription of the same set of early phase lymphokine genes, including those encoding IL-2, IL-3, IL-4, IFN-$\gamma$, TNF-$\alpha$, GM-CSF and c-myc. Although their effects upon T-cell activation are indistinguishable, FK-506 and CsA bind to distinct, abundant (0.1%-0.4% of total cellular protein), cytosolic receptors termed the FK-506 binding protein (FKBP; Mr = 11,000) and cyclophilin (Mr = 17,000), respectively.

FKBP is not a lymphoid-specific protein, but is widely distributed in tissues and throughout the phyla. The FKBP protein is a member of a new class of enzymes termed peptidyl-prolyl isomerases (PPIases) which catalyze isomerization between the cis and trans forms of the Xaa-Pro bond in peptides and proteins. Rotation about the peptidyl-prolyl bond is one of the predominant slow steps involved in refolding denatured proteins in vitro. Its ubiquity and abundance suggest that FKBP has an important role in cellular physiology, perhaps in accelerating interconversion between rotational conformers of denatured or recently synthesized proteins. The PPIase activity of FKBP is inhibited by FK-506. FKBP has been purified to homogeneity from human T-lymphocytes and, more recently, from calf thymus and S. cerevisiae. The FKBPs from all three sources exhibit equivalent molecular weights as measured by SDS-PAGE, are immunologically cross-reactive, and possess PPIase activity that is inhibitable by FK-506. The PPIase activity of the yeast FKBP is significantly higher than that exhibited by the bovine and human FKBPs.

Protein sequencing of the S. cerevisiae, bovine, and human FKBPs reveals few amino acid differences between the human and bovine proteins and demonstrates that the yeast protein is homologous to the mammalian FKBPs. The high degree of amino acid conservation in FKBP from these divergent species as well as the abundance of the protein in all three organisms is another indication that FKBP has an important role in cellular physiology.

2. Brief Description of the Prior Art

FKBP has been purified to homogeneity from human T-lymphocytes from calf thymus. Siekierka et al., Nature, 341, 755-757 (1989); and Harding et al., Nature, 341, 758-760 (1989). Maki et al., Proc. Natl. Acad. Sci. USA, 87, 5440-5443 (1990) have recently reported the complete cDNA sequence of an FKBP clone isolated from a human T-cell library. The amino-terminal sequence of the human FKBP, as deduced from the nucleotide sequence, matches the reported protein sequence except for the addition of an initiator methionine in the clone.

SUMMARY OF THE INVENTION

The present invention relates to the nucleotide sequence of the cDNA encoding the human FK-506 binding protein, and to the amino acid sequence of said binding protein derived from said nucleotide sequence. In addition to providing these, it is an object of the present invention to provide a vector containing the DNA sequence encoding FKBP, to provide a host cell transformed with said vector containing said DNA sequence, and to provide a recombinant process for making FKBP by culturing said transformed host cell under conditions suitable for the expression of FKBP, and recovering FKBP.

The present invention further relates to the nucleotide sequence of the gene encoding the S. cerevisiae FK-506 binding protein, FKB1, and to the amino acid sequence of said binding protein derived from said nucleotide sequence. In addition to providing these, it is an object of the present invention to provide a vector containing the DNA sequence encoding FKB1, to provide a host cell transformed with said vector containing said DNA sequence, and to provide a recombinant process for making FKB1 by culturing said transformed host cell under conditions suitable for the expression of FKB1, and recovering FKB1.

The powerful genetic and molecular approaches available in yeast make it an especially attractive organism in which to undertake a detailed molecular analysis of FKBP. A comprehension of FKBP's function in yeast may provide insights to its function in T-cells. It has been found that by ablation of one copy of FKB1 in a diploid strain and tetrad analysis of the spores derived from the resulting heterozygote that the gene is not essential for viability and is present once per haploid genome.

The present invention also relates to a method of testing chemical compounds in order to identify those which have immunosuppressive activity capable of blocking allograft rejection by preventing T-cell activation, comprising bringing said test chemical compound into contact with FKBP or FKB1 and determining whether said test compound will bind thereto.

It is a further object of the present invention to provide purified nucleic acid probes which will enable the determination of FKBP and FKB1 expression in various species and in various tissues and cells within the selected species.

## DESCRIPTION OF THE DRAWINGS

Fig. 1.

Nucleotide sequence of the S. cerevisiae FK-506 binding protein gene and the predicted amino acid sequence. The sequence shown is 1009 bases of the 2kb genomic EcoR1 fragment containing FKB1. Numbering of the nucleotides begins at the initiating codon and residues in the 5' untranslated region are represented by negative numbers. Potential TATA boxes are underlined. The predicted amino acid sequence is shown below the nucleotide sequence.

Fig. 2.

Nucleotide sequence of the cDNA encoding the human FK-506 binding protein. Numbering commences at the initiator methionine with 5' untranslated sequences represented by negative numbers. The derived amino acid sequence is shown below the nucleotide sequence.

## DETAILED DESCRIPTION OF THE INVENTION

### Libraries -

The yeast genomic library in λgt10 was constructed by inserting EcoRI-digested yeast DNA into the EcoRI site of λgt10 and has been previously described by Phizicky et al. in J. Biol. Chem., 261, 2978-2986 (1986). The JURKAT cDNA library in λgt10 was purchased from Clontech.

### Oligonucleotide Probes -

Degenerate, oligonucleotide probes were synthesized on a Milligen Scientific Cyclone Model 8400 DNA Synthesizer. For the probes used to isolate the yeast FKBP gene, deoxyinosines were substituted in some positions of base ambiguity. Two non-overlapping 29mers were synthesized in order to screen the yeast genomic library. The oligonucleotide sequences as well as the yeast FKBP amino acid sequences they were derived from are shown below:

```
ASP GLY ALA THR PHE PRO LYS THR GLY ASP
CTA CCI CGI TGI AAA GGI TTT TGI CCI CT
 G           G       C
```

```
ASN ILE GLY VAL GLY GLN VAL ILE LYS GLY
TTA TAI CCI CAI CCI GTT CAI TAI TTT CC
 G               C           C
```

One degenerate, deoxyinosine-containing 38mer oligonucleotide probe was synthesized in order to screen the JURKAT cDNA library. Deoxyinosines were used to probe T,C,A,G ambiguities and some T,C ambiguities while thymidines were used to probe some A,G ambiguities according to the recommendations outlined by Martin et al. in Nucl. Acids Res., 13, 8927-8938 (1985). The oligonucleotide synthesized and the corresponding human FKBP amino acid sequence it was derived from is shown below:

```
HIS TYR THR GLY MET LEU GLU ASP GLY LYS LYS PHE ASP
CAT TAT ACI GGI ATG TTI GAI GAT GGI AAI AAI TTT GA
    C   C                           C
```

**Screening Libraries -**

The yeast genomic and JURKAT cDNA libraries were plated on E. coli strain C600 hf1A. To screen the yeast library, four 150 mm petri dishes containing 4,000 recombinant phage per dish were plated. Plate replicas were made on Nytran membranes (Schleicher and Schuell). Denaturation, renaturation, and baking of the phage DNA to the filters were performed according to the manufacturer's instructions. The filters were prehybridized and hybridized to a 32P-labeled oligonucleotide probe according to the methods described by Itoh et al. in J. Biol. Chem., 262, 3132-3135 (1986). The filters were prehybridized for four hours at 45°C in a solution containing 5X SSC, 10X Denhardt's, 50 mM sodium phosphate, and 0.1% SDS. The 32P-labeled oligonucleotide probe (1 ng/ml; 4 x 107 cpm/pmol) was added to the prehybridization solution and the filters hybridized at 45°C for sixteen hours. The filters were washed four times at twenty minutes per wash at room temperature in a washing solution containing 5X SSC, 0.1% SDS, and 50 mM sodium phosphate. This was followed by an additional wash at 45°C for one minute in the washing solution. The wet filters were wrapped in Saran Wrap and allowed to expose X-ray film (XAR-5; Eastman Kodak, Rochester, N.Y.) for 36 hours at -80°C using intensifying screens. To isolate the human FKBP gene, ten 150 mm plates containing 30,000 plaques per plate of the JURKAT cDNA library were screened.

Prehybridization and hybridization conditions were performed as described above for the isolation of the yeast gene except that the one minute wash at 45°C was omitted.

**DNA Sequencing of Phage DNA Inserts -**

Positive phage were plaque-purified and a plug containing a single plaque was placed in 1 ml of SM containing 20 ml of chloroform. After elution of the phage from the plug for 1 hour, the phage were allowed to infect E. coli and plated to near-confluent lysis in LB-top agarose on LB-agarose plates supplemented with 1mM MgSO4. After bacterial lysis, SM (5 ml) was added to each plate and the plates were shaken for two hours. The SM was transferred to a tube and debris removed by centrifugation. To the supernatant, RNAse and DNAse were added to 1 mg/ml, 4 ml of 20% PEG 8000/2M NaCl were added, and the tube incubated on ice for 1.5 hr. Phage were precipitated by centrifugation (10,000 rpm/ 20 min.) in a Beckman JA-20 rotor. The pellet was resuspended in 0.5 ml SM and 5 ml of 10% SDS, 12.5 ml of 0.2 M EDTA (pH 8.0), and 2.6 ml of 10 mg/ml Proteinase K were added. After incubation at 68°C for 1 hr, protein was removed by phenol/chloroform/isoamyl alcohol extraction. Phage DNA was precipitated upon the addition of 500 ml isopropanol. After resuspension of the phage DNA, it was digested with EcoRI to remove the insert. The insert was subcloned into pUC19. Sequencing of both DNA strands by the dideoxy method of Biggin et al., Proc. Natl. Acad. Sci. USA, 80, 3963-3965 (1983) was performed directly from denatured plasmid miniprep DNA.

**Yeast Strains and Methods -**

Yeast were grown on media (YPD: yeast extract, peptone, dextrose; SD: synthetic minimal) previously described by Sherman et al., Methods in Yeast Genetics, Cold Spring Harbor, New York: Cold Spring Harbor Laboratory (1979). For Southern analysis, yeast DNA was isolated as described by Winston et al., Meth. Enzymol., 65, 211-228 (1983). The diploid strain YFK016 used to construct the gene disruption is the product of a cross between the haploid strains YPH54 and YPH98, Sikorski and Hieter, Genetics, 122, 19-27 (1989). Yeast protein extracts were prepared from 6 ml early stationary cultures grown in YPD. Cells were disrupted by glass beads in 0.3 ml of buffer containing 150 mM Tris (pH 7.5), 10 mM MgCl2, 1 mM DTT, 10% glycerol, 1 mM PMSF, 3 mg/ml benzamidine, 1 mg/ml aprotinin, and 1 mg/ml leupeptin. Insoluble material was removed by centrifugation for 15 min in a microfuge. The supernatant was assayed using the [3H]FK-506 binding assay previously described in Siekierka et al., Nature, 341, 755-757 (1989).

**Sequence Analysis** -

Sequence analysis of the FKBPs was performed using the programs of the GCG package described in Deveraux et al., Nucl. Acids Res., 12, 387-395 (1984).

**Isolation of the Yeast FKBP Gene**

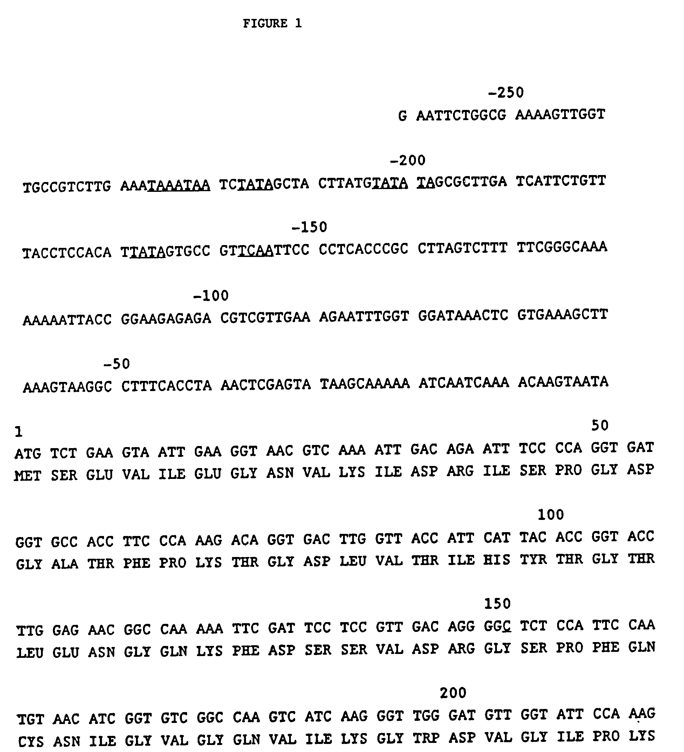

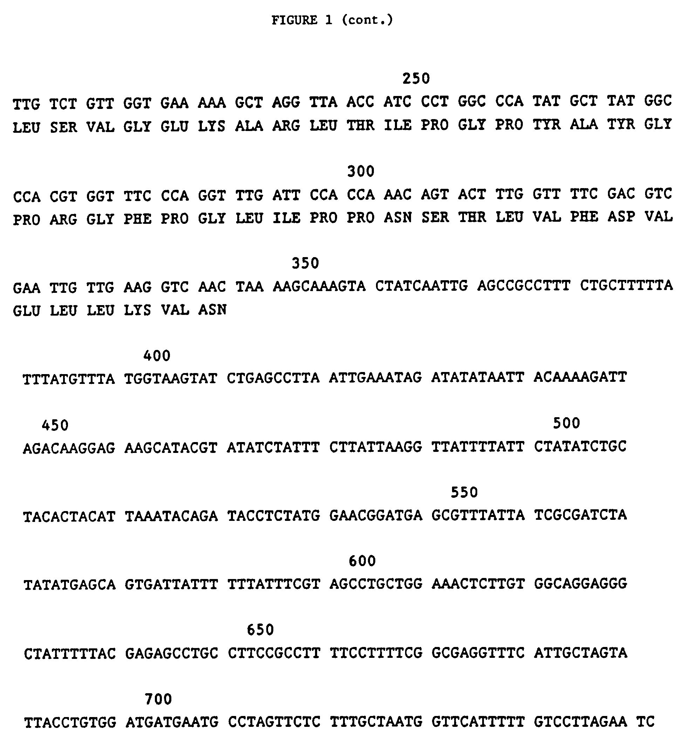

The amino acid sequence of the S. cerevisiae FKBP was utilized to design and synthesize two non-overlapping oligonucleotide probes for screening a yeast genomic DNA library in λgt10. Due to the codon degeneracy specified by the amino acid sequence, each of the two oligonucleotides was of 8-fold complexity and contained six inosines. 16,000 recombinant phage were screened and five plaques hybridized to both oligonucleotide probes through several rounds of screening. All five phage contained a 2 kb insert when digested with EcoR1. Sequencing of approximately 1 kb of the 2 kb insert revealed an open reading frame (ORF) encoding a 114 amino acid protein of 12,150 Daltons, a mass in agreement with the molecular weight of the purified yeast FKBP as determined by SDS-PAGE. All amino acids are present at least once in the sequence; however, the protein is relatively high in glycine and proline content (14% and 8.8% by number, respectively). The authenticity of the clone is verified by the fact that the amino acid sequence is entirely consistent with the seventy-seven amino acids obtained from peptide sequencing. The sequence is shown in Figure 1 of the drawings.

Compatible with the observation that the FKBP is an abundant protein are the facts that the initiator methionine is in an excellent context for translation initiation, having an adenine at -3, and that there is a thymidine at +6, a consensus residue among highly expressed yeast genes. Furthermore, from an analysis of codon usage, the codon bias index of FKB1 was calculated to be 0.71, a value demonstrating highly biased codon representation and predicting a high level of mRNA in the cytoplasm. Promoter analysis reveals several potential TATA boxes, at -227, -218, -204, -169, and -32, suggesting that, like most yeast genes, FKB1 has multiple transcription start sites.

**Isolation of the Human FKBP Gene**

From the amino acid sequence of the human FKBP, a single 38mer oligonucleotide was designed and synthesized in order to screen a JURKAT cDNA library in λgt10. Because of the codon degeneracy of the amino acids in the sequence, the oligonucleotide was 8-fold complex and included seven inosines. 300,000 recombinant phage were screened and one plaque was purified that reproducibly hybridized to the probe. The positive phage contained a 1.5 kB insert that was subcloned into pUC19, sequenced and found to contain a partial cDNA; 1418 nucleotides matched the sequence reported by Maki et al., Proc. Natl. Acad. Sci. USA, 87, 5440-5443 (1990). Using the amino acid sequence from Siekierka et al., Nature, 341, 755-757 (1989), the remaining 12 amino acids and an initiator methionine were PCR'd onto the end of the gene. The sequence of the human FKBP gene matches that reported by Maki et al. (supra) and contains an ORF of 108 amino acids. The authenticity of the clone is verified by the fact that 36 residues of amino acid sequence data match the sequence found in the ORF. The human FKBP is average in terms of proline content but, like the yeast protein, is relatively high in glycine content (12% by number). The sequence is shown in Figure 2 of the drawings. The human FKBP shares 57% identity and 75% similarity with the yeast FKBP.

**Construction of the Human FK506 Binding Protein Gene Fused to an E. Coli Expression Vector**

A partial cDNA clone for the human FK506 binding protein gene was obtained by screening a JURKAT cDNA library with oligonucleotides. Coding sequence for eleven N-terminal amino acids were missing and were supplied to the clone by a chemically synthesized 40bp fragment with a 5'-NcoI site and a 3'-NcoI-complementary overhang. DNA sequence information for this fragment was derived from the known N-terminal amino acid sequence of FKBP. The sequence of the fragment is:

```
      -NcoI-      10              20             30    -NcoI complementary
ATATTCCATGGGAGTGC AGGTGGAAAC CATCTCCCCA GGA
TATAAGGTACCCTCACG TCCACCTTTG GTAGAGGGGT CCTGTAC
         M  G  V  Q    V  E  T    I  S  P    G
```

A 308 bp NcoI-BamHI fragment containing the FKBP coding sequences was cloned from the partial FKBP cDNA clone described above by polymerase chain reaction (PCR) techniques using the following primers:

```
            -NcoI-40              50
        5'-TAACCATGGAC GCACCTTCC C-3'
                                -BamHI-  ,
            3'-G AAGATTTTGA CCTTACTCCTAGGTAAT 5'
                310              320
```

To obtain the complete gene for expression vector cloning, the PCR-generated DNA fragment was digested with NcoI, ligated to the synthetic DNA fragment, digested with NcoI and BamHI, and cloned into a pBluescribe derivative. Site directed mutagenesis was performed to repair base 34 (a C to G change) in order to restore the specified amino acid sequence to match that of the wild-type protein.

The 329 bp NcoI-BamHI fragment containing the entire open reading frame for FKBP was subcloned between the NcoI and BamHI sites of pET3d (originally pET8c). The plasmid was transformed into E. coli BL21(DE3) cells. Cells containing the plasmid produce the human FKBP. A description of the pET series of vectors and further details of their use can be found in the following references: Rosenberg et al. (1987) Gene, 56: 125; Studier et al. (1986) J. Mol. Biol. 189:113; and Studier et al (1990) Methods in Enzymology, 185:60.

The recombinant human FKBP protein was fully functional in binding [$^3$H] FK-506 and exhibited peptidyl-prolyl cis-trans isomerase activity.

## Claims

1. A DNA sequence coding for the human FK-506 binding protein.

2. A DNA sequence as shown in Figure 2 coding for the human FK-506 binding protein.

3. The human FK-506 binding protein in substantially biologically pure form.

4. The human FK-506 binding protein having the amino acid sequence as shown in Figure 2.

5. A vector containing the DNA sequence of Claim 2.

6. A microbial host transformed by the vector of Claim 5 containing the DNA sequence coding for the human FK-506 binding protein.

7. A process for the preparation of the human FK-506 binding protein comprising culturing the transformed host of Claim 6 under conditions suitable for the expression of the human FK-506 binding protein, and recovering said binding protein.

8. Human FK-506 binding protein made by the process of Claim 7.

9. A DNA sequence coding for the S. cerevisiae FK-506 binding protein.

10. A DNA sequence as shown in Figure 1 coding for the S. cerevisiae FK-506 binding protein.

11. The S. cerevisiae FK-506 binding protein in substantially biologically pure form.

12. The S. cerevisiae FK-506 binding protein having the amino acid sequence as shown in Figure 1.

13. A vector containing the DNA sequence of Claim 10.

14. A microbial host transformed by the vector of Claim 13 containing the DNA sequence coding for the S. cerevisiae FK-506 binding protein.

15. A process for the preparation of the S. cerevisiae FK-506 binding protein comprising culturing the transformed host of Claim 14 under conditions suitable for the expression of the S. cerevisiae FK-506 binding protein, and recovering said binding protein.

16. S. cerevisiae FK-506 binding protein made by the process of Claim 15.

17. A method of testing chemical compounds in order to identify those which have immunosuppressive activity capable of blocking allograft rejection by preventing T-cell activation, comprising bringing said test chemical compound into contact with the human FK-506 binding protein or the S. cerevisiae binding protein and determining whether said test compound will bind thereto.

EP 0 481 673 A2

FIGURE 1

```
                                         -250
                              G AATTCTGGCG AAAAGTTGGT

                         -200
   TGCCGTCTTG AAATAAATAA TCTATAGCTA CTTATGTATA TAGCGCTTGA TCATTCTGTT

                    -150
   TACCTCCACA TTATAGTGCC GTTCAATTCC CCTCACCCGC CTTAGTCTTT TTCGGGCAAA

               -100
   AAAAATTACC GGAAGAGAGA CGTCGTTGAA AGAATTTGGT GGATAAACTC GTGAAAGCTT

          -50
   AAAGTAAGGC CTTTCACCTA AACTCGAGTA TAAGCAAAAA ATCAATCAAA ACAAGTAATA
```

```
1                                                                 50
ATG TCT GAA GTA ATT GAA GGT AAC GTC AAA ATT GAC AGA ATT TCC CCA GGT GAT
MET SER GLU VAL ILE GLU GLY ASN VAL LYS ILE ASP ARG ILE SER PRO GLY ASP

                                                     100
GGT GCC ACC TTC CCA AAG ACA GGT GAC TTG GTT ACC ATT CAT TAC ACC GGT ACC
GLY ALA THR PHE PRO LYS THR GLY ASP LEU VAL THR ILE HIS TYR THR GLY THR

                                                150
TTG GAG AAC GGC CAA AAA TTC GAT TCC TCC GTT GAC AGG GGC TCT CCA TTC CAA
LEU GLU ASN GLY GLN LYS PHE ASP SER SER VAL ASP ARG GLY SER PRO PHE GLN

                                           200
TGT AAC ATC GGT GTC GGC CAA GTC ATC AAG GGT TGG GAT GTT GGT ATT CCA AAG
CYS ASN ILE GLY VAL GLY GLN VAL ILE LYS GLY TRP ASP VAL GLY ILE PRO LYS
```

9

FIGURE 1 (cont.)

250
TTG TCT GTT GGT GAA AAA GCT AGG TTA ACC ATC CCT GGC CCA TAT GCT TAT GGC
LEU SER VAL GLY GLU LYS ALA ARG LEU THR ILE PRO GLY PRO TYR ALA TYR GLY

300
CCA CGT GGT TTC CCA GGT TTG ATT CCA CCA AAC AGT ACT TTG GTT TTC GAC GTC
PRO ARG GLY PHE PRO GLY LEU ILE PRO PRO ASN SER THR LEU VAL PHE ASP VAL

350
GAA TTG TTG AAG GTC AAC TAA AAGCAAAGTA CTATCAATTG AGCCGCCTTT CTGCTTTTTA
GLU LEU LEU LYS VAL ASN

400
TTTATGTTTA TGGTAAGTAT CTGAGCCTTA ATTGAAATAG ATATATAATT ACAAAAGATT

450                                                        500
AGACAAGGAG AAGCATACGT ATATCTATTT CTTATTAAGG TTATTTTATT CTATATCTGC

550
TACACTACAT TAAATACAGA TACCTCTATG GAACGGATGA GCGTTTATTA TCGCGATCTA

600
TATATGAGCA GTGATTATTT TTTATTTCGT AGCCTGCTGG AAACTCTTGT GGCAGGAGGG

650
CTATTTTTAC GAGAGCCTGC CTTCCGCCTT TTCCTTTTCG GCGAGGTTTC ATTGCTAGTA

700
TTACCTGTGG ATGATGAATG CCTAGTTCTC TTTGCTAATG GTTCATTTTT GTCCTTAGAA TC

FIGURE 2

```
                                                -10
                                        GCGTC CGCCGCCGCC
1
ATG GGA GTG CAG GTG GAA ACC ATC TCC CCA GGA GAC GGG CGC ACC
MET GLY VAL GLN VAL GLU THR ILE SER PRO GLY ASP GLY ARG THR


      50
TTC CCC AAG CGC GGC CAG ACC TGC GTG GTG CAC TAC ACC GGG ATG
PHE PRO LYS ARG GLY GLN THR CYS VAL VAL HIS TYR THR GLY MET


         100
CTT GAA GAT GGA AAG AAA TTT GAT TCC TCC CGG GAC AGA AAC AAG
LEU GLU ASP GLY LYS LYS PHE ASP SER SER ARG ASP ARG ASN LYS


            150
CCC TTT AAG TTT ATG CTA GGC AAG CAG GAG GTG ATC CGA GGC TGG
PRO PHE LYS PHE MET LEU GLY LYS GLN GLU VAL ILE ARG GLY TRP


               200
GAA GAA GGG GTT GCC CAG ATG AGT GTG GGT CAG AGA GCC AAA CTG
GLU GLU GLY VAL ALA GLN MET SER VAL GLY GLN ARG ALA LYS LEU


                  250
ACT ATA TCT CCA GAT TAT GCC TAT GGT GCC ACT GGG CAC CCA GGC
THR ILE SER PRO ASP TYR ALA TYR GLY ALA THR GLY HIS PRO GLY


                     300
ATC ATC CCA CCA CAT GCC ACT CTC GTC TTC GAT GTG GAG CTT CTA
ILE ILE PRO PRO HIS ALA THR LEU VAL PHE ASP VAL GLU LEU LEU
```

FIGURE 2 (cont. - pg. 2 of 3)

```
                                        350
AAA CTG GAA TGA      CA GGAATGGCCT CCTCCCTTAG CTCCCTGTTC
LYS LEU GLU OPA
                                        400
    TTGGATCTGC CATGGAGGGA TCTGGTGCCT CCAGACATGT GCACATGAGT
                                        450
    CCATATGGAG CTTTTCCTGA TGTTCCACTC CACTTTGTAT AGACATCTGC
                                        500
    CCTGACTGAA TGTGTTCTGT CACTCAGCTT TGCTTCCGAC ACCTCTGTTT
                                        550
    CCTCTTCCCC TTTCTCCTCG TATGTGTGTT TACCTAAACT ATATGCCATA
                                        600
    AACCTCAAGT TATTCATTTT ATTTTGTTTT CATTTGGGG TGAAGATTCA
                                        650
    GTTTCAGTCT TTTGGATATA GGTTTCCAAT TAAGTACATG GTCAAGTATT
                                        700
    AACAGCACAA GTGGTAGGTT AACATTAGAA TAGGAATTGG TGTTGGGGGG
                                        750
    GGGGTTTGCA AGAATATTTT ATTTTAATTT TTTGGATGAA ATTTTTATCT
                                        800
    ATTATATATT AAACATTCTT GCTGCTGCGC TGCAAAGCCA TAGCAGATTT
                                        850
    GAGGCGCTGT TGAGGACTGA ATTACTCTCC AAGTTGAGAG ATGTCTTTGG
                                        900
    GTTAAATTAA AAGCCCTACC TAAAACTGAG GTGGGGATGG GGAGAGCCTT
                                        950
    TGCCTCCACC ATTCCCACCC ACCCTCCCCT TAAACCCTCT GCCTTTGAAA
                                        1000
    GTAGATCATG TTCACTGCAA TGCTGGACAC TACAGGTATC TGTCCCTGGG
                                        1050
    CCAGCAGGGA CCTCTGAAGC CTTCTTTGTG GCCTTTTTTT TTTTTCATCC
```

FIGURE 2 (cont. Pg 3 of 3)

```
                                    1100
TGTGGTTTTT CTAATGGACT TTCAGGAATT TTGTAATCTC ATAACTTTCC
                                    1150
AAGCTCCACC ACTTCCTAAA TCTTAAGAAC TTTAATTGAC AGTTTCAATT
                                    1200
GAAGGTGCTG TTTGTATACT TAACACCCAG TGAAAGCCCA GCCATCATGA
                                    1250
CAAATCCTTG AATGTTCTCT TAAGAAAATG ATGCTGGTCA TCGCAGCTTC
                                    1300
AGCATCTCCT GTTTTTTGAT GCTTGGCTCC CTCTGCTGAT CTCAGTTTCC
                                    1350
TGGCTTTTCC TCCCTCAGCC CCTTCTCACC CCTTTGCTGT CCTGTGTAGT
                                    1400
GATTTGGTGA GAAATCGTTG CTGCACCCTT CCCCCAGCAC CATTTATGAG
                                    1449
TCTCAAGTTT TATTATTGCA ATAAAAGTGC TTTATGCCCG
```